(19) Europäisches Patentamt
European Patent Office
Office européen des brevets

(11) EP 1 064 246 B1

(12) **EUROPÄISCHE PATENTSCHRIFT**

(45) Veröffentlichungstag und Bekanntmachung des Hinweises auf die Patenterteilung:
**23.06.2004 Patentblatt 2004/26**

(21) Anmeldenummer: **99939843.1**

(22) Anmeldetag: **25.02.1999**

(51) Int Cl.[7]: **C07C 45/38**, C07C 45/39, C07C 47/127, C07C 47/04

(86) Internationale Anmeldenummer:
**PCT/EP1999/001215**

(87) Internationale Veröffentlichungsnummer:
**WO 1999/047481 (23.09.1999 Gazette 1999/38)**

(54) **VERFAHREN ZUR HERSTELLUNG VON CARBONYLVERBINDUNGEN**
METHOD FOR PRODUCING CARBONYL COMPOUNDS
PROCEDE DE PRODUCTION DE COMPOSES CARBONYLES

(84) Benannte Vertragsstaaten:
**BE DE FR**

(30) Priorität: **16.03.1998 DE 19811288**

(43) Veröffentlichungstag der Anmeldung:
**03.01.2001 Patentblatt 2001/01**

(73) Patentinhaber: **BASF AKTIENGESELLSCHAFT**
**67056 Ludwigshafen (DE)**

(72) Erfinder:
- **DEMUTH, Dirk**
  **69226 Nussloch (DE)**
- **FETZER, Thomas**
  **D-67346 Speyer (DE)**
- **PRATSCH, Volker**
  **D-68307 Mannheim (DE)**
- **WENDRICH, Thomas**
  **D-67316 Carlsberg (DE)**
- **MENIG, Helmuth**
  **D-67159 Friedelsheim (DE)**
- **WAMBACH, Ludwig**
  **D-68723 Schwetzingen (DE)**
- **HARTH, Klaus**
  **D-67317 Altleiningen (DE)**

(74) Vertreter: **Isenbruck, Günter, Dr. et al**
**Isenbruck, Bösl, Hörschler, Wichmann, Huhn, Patentanwälte**
**Theodor-Heuss-Anlage 12**
**68165 Mannheim (DE)**

(56) Entgegenhaltungen:
**EP-A- 0 566 924**
**DE-A- 19 654 046**
**DE-A- 19 654 054**
**FR-A- 2 319 613**
**US-A- 3 948 997**
**US-A- 4 233 246**
**US-A- 4 555 583**

- **PATENT ABSTRACTS OF JAPAN vol. 007, no. 147 (C-173), 28. Juni 1983 & JP 58 059933 A (MITSUI TOATSU KAGAKU KK), 9. April 1983**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 064 246 B1

## Beschreibung

Die Erfindung betrifft ein neues Verfahren zur Herstellung von Carbonylverbindungen durch Gasphasenoxidation von Alkoholen mit Sauerstoff enthaltendem Gas in Gegenwart eines Katalysatorsystems aus einem im wesentlichen phosphorfreien und mindestens einem phosphorhaltigen Kupfer und/oder Silber enthaltenden Katalysator.

Verfahren zur Herstellung von Carbonylverbindungen durch Gasphasenoxidation an Kupfer oder Silber-Katalysatoren in Gegenwart flüchtiger Phosphorverbindungen sind aus dem Stand der Technik bekannt.

So ist in der EP-A 007 570 ein Verfahren zur Herstellung von Glyoxal durch Gasphasenoxidation von Ethylenglykol mit Sauerstoff an einem Kupfer enthaltenden Oxidationskatalysator in Gegenwart von unter Reaktionsbedingungen flüchtigen Phosphorverbindungen beschrieben, bei dem die Phosphormenge von 1 bis 100 ppm, bezogen auf eingesetztes Ethylenglykol, mit den Ausgangsverbindungen zugegeben wird. Bei diesen Verfahren werden unbefriedigende Glyoxalausbeuten bis 70 Mol-%, bezogen auf umgesetztes Ethylenglykol, erzielt.

Nach den Verfahren der US-A 4 282 374 und der US-A 4 503 261 erhält man bei der Gasphasenoxidation von Ethylenglykol an Kupferkatalysatoren oder an einem Schichtkatalysator aus Kupfer- und Silberkristallen vorteilhafte Ergebnisse hinsichtlich der Lebensdauer der Katalysatoren und der Glyoxalausbeute, wenn man die Reaktion in Gegenwart einer flüchtigen Phosphorverbindung durchführt, wobei die Phosphormenge (berechnet P), bezogen auf die Gewichtsmenge an Ethylenglykol, 1 bis 100 ppm bzw. 0,5 bis 20 ppm beträgt und mit den Ausgangsverbindungen vor der Katalysatorschüttung zugegeben wird. Bei diesen Verfahren hat sich jedoch bei längerer Betriebsdauer herausgestellt, daß die Glyoxalausbeute und Produktreinheit mit der Versuchsdauer zunehmend schlechter werden. Dieser Nachteil ist auf eine vermehrte Bildung von Formaldehyd und von $CO/CO_2$ zurückzuführen.

In der EP-B 0 271812 wird für die Herstellung von Carbonylverbindungen wie Glyoxal, eine Gasphasenoxidation von Alkoholen mit einem Sauerstoff enthaltenden Gas in Gegenwart von Kupfer oder Silber enthaltenden Katalysatoren und einer unter den Reaktionsbedingungen flüchtigen Phosphorverbindung vorgeschlagen, bei der die Phosphorverbindung in einer Portion in einer Menge von weniger als 0,5 ppm, bezogen auf die Gewichtsmenge an eingesetztem Alkohol und berechnet als Phosphor, in dem gasförmigen Ausgangsgemisch vor der Umsetzung an dem Katalysator zugemischt wird. Nach dem in der EP-B 0 271 812 beschriebenen Verfahren wird Glyoxal in Ausbeuten bis 80 Mol-% erhalten.

Weiterhin ist aus der DE-A 19 23 048 ein Verfahren zur Herstellung von Carbonylverbindungen durch Gasphasenoxidation von Hydroxyverbindungen an Katalysatorschüttungen bestehend aus 1 bis 5 Gew.-% Phosphor enthaltenden Kupfer-Phosphor-Katalysatoren bekannt. Bei diesem Verfahren werden unbefriedigende Glyoxalbeuten erzielt.

Die WO-A 9828250 und WO-A 9828251 betreffen Verfahren zu Herstellung von Carbonylverbindungen durch Gasphasen oxidation der entsprechenden Alkohole mit einem Sauerstoff enthalten den Gas in Gegenwart von Kupfer und/oder Silber enthaltenden. Katalysatoren und Phosphorverbindungen, die unter den jeweiligen Reaktionsbedingungen flüchtig sind. Diese Phosphorverbindungen werden entwerder dicht in die Katalysatorschüttung oder zum einen Teil in die Katalysatorschüttung und zum zuderen Teil in die gasförmige Ausgangsgemischung eingetragen.

Die vorstehenden Verfahren des Standes der Technik weisen den Nachteil einer unbefriedigenden Ausbeute auf Bei dem bekannten Verfahren fällt Glyoxal als wäßrige, mit Glykolaldehyd, Formaldehyd und organischen Säuren verunreinigte Lösung an. Weitere unerwünschte Nebenprodukte sind die anfallenden Verbrennungsprodukte CO, $CO_2$ und $H_2O$. Als Folge der Nebenprodukte weisen die bekannten Verfahren zusätzlich den Nachteil unbefriedigender Katalysatorstandzeiten auf.

Anteile von Formaldehyd im Glyoxal sind für viele Anwendungszwecke des Glyoxals zudem wegen der toxikologischen Eigenschaften und der hohen Reaktivität des Formaldehyds höchst unerwünscht. Da man Formaldehyd nur mit einem erheblichen Aufwand und unter Inkaufnahme von Ausbeuteverlusten aus dem rohen Glyoxal entfernen kann, etwa durch Behandlung mit Wasserdampf oder durch chemische Umsetzung, war nach einem Verfahren zu suchen, das es gestattet, Glyoxal durch katalytische Gasphasenoxidation von Ethylenglykol auch bei langen Betriebszeiten unter weitgehender Vermeidung der Bildung störender Nebenprodukte herzustellen.

Es wurde nun gefunden, daß man bei der Herstellung von Carbonylverbindungen der Formel

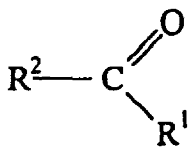

(I)

in der $R^1$ ein Wasserstoffatom oder einen Alkylrest mit 1 bis 3 C-Atomen, $R^2$ ein Wasserstoffatom, einen unsubstituierten oder mit $C_1$- bis $C_3$-Alkyl ein- bis dreifach substituierten $C_2$- bis $C_4$-Alkenylrest oder einen Rest der Formel

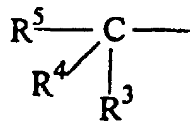

(II)

in der $R^3$ ein Wasserstoffatom oder zusammen mit $R^4$ ein Sauerstoffatom, $R^4$ den Rest $OR^6$ oder zusammen mit $R^3$ ein Sauerstoffatom, $R^5$ ein Wasserstoffatom, einen unsubstituierten oder mit $C_1$- bis $C_3$-Alkyl ein bis dreifach substituierten $C_2$bis $C_4$-Alkenylrest, einen Alkylrest mit 1 bis 8 C-Atomen oder einen Cyclohexyloder Cyclopentylrest und $R^6$ einen Alkylrest mit 1 bis 4 C-Atomen, einen Cyclohexyl- oder Cyclopentylrest oder einen Rest der Formel $-CH_2-CHO$ oder $-CH_2-CH_2-O-CH_2-CHO$ bedeuten, durch Gasphasenoxidation von Methanol oder Alkoholen der Formel

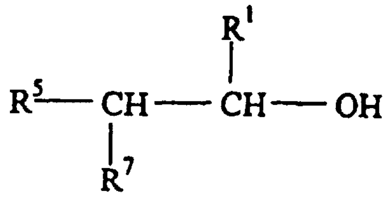

(III)

in der $R^1$ und $R^5$ die oben angegebene Bedeutung haben und $R^7$ ein Wasserstoffatom oder einen Rest $OR^8$ und $R^8$ ein Wasserstoffatom, einen Alkylrest mit 1 bis 4 C-Atomen, einen Cyclohexyl- oder Cyclogentylrest oder einen Rest der Formel $-CH_2-CH_2-OH$ oder $-CH_2-CH_2-O-CH_2-CH_2-OH$ bedeutet, mit einem Sauerstoff enthaltenden Gas in Gegenwart von Kupfer und/oder Silber enthaltenden Katalysatoren und gegebenenfalls einer unter Reaktionsbedingungen flüchtigen Phosphorverbindung in einer Menge, so daß die Phosphormenge (berechnet als P), bezogen auf die Gewichtsmenge an eingesetztem Alkohol, bis zu 20 ppm beträgt, dadurch gekennzeichnet, daß eine Katalysatorschüttung aufgebaut an einem Katalysatorsystem aus einem im wesentlichen phosphorfreien Kupfer und/oder Silberenthaltenden Katalysator mit einem Phosphorgehalt von 0 bis 400 ppm und mindestens einem phosphorhaltigen Kupfer und/oder Silber enthaltenden Katalysator mit einem Phosphorgehalt von 1000 bis 10000 ppm eingesetzt wird.

Nach dem neuen Verfahren wird Glyoxal aus Ethylenglykol im Dauerbetrieb in hoher Ausbeute und Reinheit und bei deutlich reduziertem Formaldehydanteil erhalten. Das neue Verfahren ist zudem in Großanlagen technisch einfach umsetzbar.

In den Alkoholen der Formel III bedeuten Alkylreste z.B. Methyl-, Ethyl-, Propyloder Butylreste. Bei dem erfindungsgemäßen Verfahren gehen die endständigen Hydroxylgruppen in Aldehydgruppen und die sekundären Hydroxylgruppen in Ketogruppen über.

Beispielsweise seien die folgende Ausgangsverbindungen der Formel III genannt: $HO—CH_2—CH_2—OH$, $H_3COCH_2—CH_2OH$, $CH_3—CH_2OH$, $C_2H_5OCH_2—CH_2OH$, $H_3C—CH(OH)—CH_2—OH$, $C_2H_5—CH(OH)—CH_2OH$,

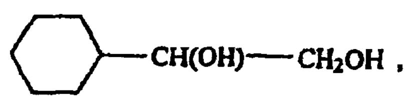

$HO—(CH_2)_2—O—(CH_2)_2—OH$ ,

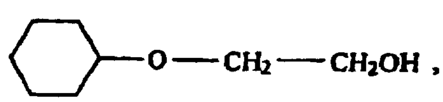

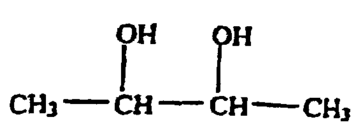

Die Gasphasenoxidation des Alkohols mit dem Sauerstoff enthaltenden Gas an den Kupfer und/oder Silber

enthaltenden Katalysatoren führt man auf an sich bekannte Weise, z.B. bei Temperaturen von 225 bis 500°C, durch.

Als Kupfer und/oder Silber enthaltende Katalysatoren sind beispielsweise metallisches Kupfer oder Silber, kupferhaltige oder silberhaltige Legierungen oder Verbindungen mit Metallen oder Nichtmetallen geeignet, die zusätzlich Phosphor enthalten können bzw. im Fall der phosphorhaltigen Katalysatoren Phosphor enthalten, wie Kupferphosphide, Kupferbronzen oder Legierungen des Kupfers mit Silber und/oder Gold, Kupfererze wie Malachit und Kupfer- oder Silberverbindungen, die während der Reaktion ganz oder teilweise zu Kupfer oder Silber reduziert werden können, wie Kupfer(I)oxid, Silber(I)oxid, Kupfer(II)oxid, und Verbindungen, die beim Erhitzen in Kupferoxide übergehen, wie Kupfernitrat und Kupferacetat. Ferner sind auch Kupferphosphat und Kupferantimonat geeignet. Den kupferhaltigen Verbindungen können zusätzlich noch andere Metall- oder Nichtmetalloxide wie die Oxide von Zink, Chrom, Antimon, Zinn, Wismut beigemischt sein.

Die im wesentlichen phosphorfreien Kupfer und/oder Silber enthaltenden Katalysatoren im Sinne der vorliegenden Anmeldung weisen einen Phosphorgehalt von 0 bis 400 ppm, vorzugsweise von 150 bis 400 ppm entsprechend 0,015 bis 0,04 Gew.-%, und besonders bevorzugt von 150 bis 250 ppm, entsprechend 0,015 bis 0,025 Gew.-% auf und legen bevorzugt als Legierung vor.

Ein für die Verwendung in dem erfindungsgemäßen Verfahren besonders bevorzugter im wesentlichen phosphorfreier Kupfer und/oder Silber enthaltender Katalysator ist beispielsweise das nach DIN 1708 (Kathoden und Gußformate) mit SF-Cu bezeichnete Kupfer der Werkstoffnummer 2.0090, das beispielsweise von der Norddeutschen Affinerie AG, Hamburg, vertrieben wird.

Als phosphorhaltiger Kupfer und/oder Silber enthaltender Katalysator wird in dem erfindungsgemäßen Verfahren ein Katalysator mit einem Phosphorgehalt von 1000 bis 10.000 ppm, entsprechend 0,1 bis 1 Gew.-% eingesetzt. Als phosphorhaltiger Kupfer und/oder Silber enthaltende Katalysator findet bevorzugt eine Kupfer-Phosphorlegierung mit einem Phosphorgehalt von 1000 bis 10.000 ppm Verwendung, die beispielsweise durch Schmelzen von Kupferkathoden grade 1 ASTM B 170 und nach DIN 17 657 mit V-CuP10 bezeichneten Kupfer-Phosphorlegierung in geeigneten Mengenverhältnissen hergestellt werden kann. Der phosphorhaltige Katalysator kann beispielsweise von den Wieland Werken AG, Ulm, bezogen werden.

Die im wesentlichen phosphorfreie als auch die phosphorhaltige kupfer- und/oder silberhaltige katalytische Masse kann auch auf einem inerten Träger aufgebracht oder gegebenenfalls mit einem inerten Material verdünnt werden. Der Katalysator kann gegebenenfalls auch vor dem Einsatz einer reduzierenden Behandlung unterworfen werden.

Bevorzugt sind Katalysatoren, die keine große innere Oberfläche besitzen, beispielsweise solche mit einer Oberfläche von unter 50 $m^2$ pro g. Besonderes technisches Interesse haben im wesentlichen phosphorfreies als auch phosphorhaltiges metallisches Kupfer oder Silber und Legierungen, die Kupfer oder Silber als einen wesentlichen Bestandteil enthalten. Man wendet sie z.B. in Form von Drehspänen, Drahtgeweben, Gasen oder auch als Trägerkatalysatoren mit einem z.B. oberflächenarmen, inerten Träger an.

Die in dem erfindungsgemäßen Verfahren aus dem im wesentlichen phosphorfreien und mindestens einem phosphorhaltigen Kupfer und/oder silberhaltigen Katalysator aufgebaute Katalysatorschüttung kann den im wesentlichen phosphorfreien und den phosphorhaltigen Katalysator in verschiedenen räumlichen Anordnungen enthalten; das heißt die Schüttung kann verschieden strukturiert sein.

So kann die gesamte Katalysatorschüttung aus einer homogenen Mischung des im wesentlichen phosphorfreien und des phosphorhaltigen Kupfer und/oder Silber enthaltenden Katalysatos im Gewichtsverhältnis von 100:1 1 bis 1:100, bevorzugt 20:1 1 bis 1:10, besonders bevorzugt 10:1 bis 1:1 bestehen.

Es ist jedoch auch möglich, für die oberen 0,1 bis 50 % Gesamthöhe der Katalysatorschüttung, bevorzugt die oberen 0,1, 10 oder 20 %, besonders bevorzugt die oberen 30 bis 35 % Gesamthöhe, nach dem Reaktorkopf den im wesentlichen phosphorfreien Kupfer und/oder Silber enthaltenden Katalysator zu verwenden und die verbleibende Katalysatorschüttung aus einer homogenen Mischung des im wesentlichen phosphorfreien und des phosphorhaltigen Kupfers und/oder Silber enthaltenden Katalysators im Gewichtsverhältnis von 100:1 bis 1:100, bevorzugt 20:1 bis 1: 10, besonders bevorzugt 10:1 bis 1:1, bestehen. Bei dieser bevorzugten Strukturierung der Katalysatorschüttung wird der Teil der Schüttung, der nach dem Hot Spot liegt, aus der homogenen Mischung von im wesentlichen phosphorfreien und phosphorhaltigen Kupfer und/oder Silber enthaltenden Katalysator gebildet.

Weiterhin kann die Katalysatorschüttung im Bereich der oberen 0,1 bis 50 % der Gesamthöhe der Katalysatorschüttung, besonders bevorzugt im Bereich der oberen 1 bis 35 % der Katalysatorschüttung, nach dem Reaktorkopf aus dem wesentlichen phosphorfreien Kupfer und/oder Silber enthaltenden Katalysator bestehen und der phosphorhaltige Katalysator macht die restliche Katalysatorschüttung aus. Bei dieser Ausführungsform der Katalysatorschüttung besteht der Bereich der Schüttung nach dem Hot Spot aus dem phosphorhaltigen Katalysator.

Unter Hot Spot ist der Teil der Katalysatorschüttung zu verstehen, bei dem die höchste Temperatur innerhalb des Temperaturprofils der Katalysatorschüttung auftritt. Das Temperaturprofil der Katalysatorschüttung bzw. die Lage des Hot Spots wird üblicherweise bestimmt, indem man innerhalb der Katalysatorschüttung die Temperatur gegen die Schütthöhe bestimmt. Dies kann beispielsweise durch das Einbringen einer Thermohülse mit einem beweglichen Thermoelement oder aber durch ein feststehendes Multithermoelement mit mehreren Meßpunkten in Abhängigkeit von der Schütthöhe erfolgen.

Die Verwendung der vorstehend beschriebenen strukturierten Katalysatorschüttung aus im wesentlichen phosphorfreien und phosphorhaltigen Kupfer und/oder Silber enthaltenden Katalysator hat den Vorteil, daß die Phosphordosierung technisch einfach auch für Großanlagen umsetzbar ist.

In einer weiteren Ausführungsform des erfindungsgemäßen Verfahrens kann eine unter Reaktionsbedingungen flüchtige Phosphorverbindung in einer Menge, so daß die Phosphormenge (berechnet als P), bezogen auf die Gewichtsmenge an eingesetztem Alkohol bevorzugt 0,05 bis 20 ppm, beträgt, am Reaktorkopf vor der Katalysatorschüttung oder in die Katalysatorschüttung zugegeben werden.

Die Zugabe der flüchtigen Phosphorverbindung kann in jede der vorstehend beschriebenen, verschieden strukturierten Katalysatorschüttungen erfolgen.

Als unter den Reaktionsbedingungen flüchtige Phosphorverbindungen verwendet man zweckmäßigerweise Phosphorverbindungen, die unzersetzt verdampfbarsind und mit den Komponenten des Synthesegases bei den Umsetzungsbedingungen keine Reaktion eingehen. Das sind z.B. Ester der Phosphorsäure, der phosphorigen Säure oder von Phosphonsäure, wie Trimethylphosphat, Triethylphosphat, Tri-isopropylphosphat, Tri-n-propylphosphat, Trimethylphosphit, Triethylphosphit, Triethylphosphinoxid, Methylphosphonsäurediethylester, Methylphosphonsäuredimethylester oder Ethylphosphonsäurediethylester.

Die Zugabe der Phosphormenge erfolgt in dieser Ausführungsform des erfindungsgemäßen Verfahren am Reaktorkopf vor der Katalysatorschüttung oder innerhalb der Katalysatorschüttung, bevorzugt im Bereich der oberen 0,1 bis 50 % der Gesamthöhe der Katalysatorschüttung, besonders bevorzugt, im Bereich der oberen 1 bis 35 % der Katalysatorschüttung, nach dem Reaktorkopf. Die Zugabe innerhalb der Katalysatorschüttung erfolgt bevorzugt nach dem Hot Spot.

Die Zugabe der Phosphormenge kann in einem oder mehreren, beispielsweise zwei, drei oder vier Anteilen erfolgen, wobei die Zugabe bevorzugt in zwei Anteilen von je 0,05 bis 10 ppm, besonders bevorzugt von je 0,1 bis 3 ppm, erfolgt.

Weiterhin ist es möglich die Phosphormenge in mindestens zwei Anteilen zuzugeben, wobei

a) der erste Anteil mit dem gasförmigen Ausgangsgemisch vor der Katalysatorschüttung und
b) mindestens ein weiterer Anteil innerhalb der Katalysatorschüttung, bevorzugt im Bereich der oberen 0,1 bis 50 % der Gesamthöhe der Katalysatorschüttung, besonders bevorzugt im Bereich der oberen 1 bis 35 % der Gesamthöhe der Schüttung, zugegeben wird.

Diese Ausführungsform des erfindungsgemäßen Verfahrens wird z.B. so ausgeführt, daß man ein gasförmiges Gemisch aus dem Alkohol und Wasser, wobei der Wassergehalt 0,1 bis 99 Gew.-% beträgt, zusammen mit Luft oder Sauerstoff in einer Menge zwischen 0,5 und 2,0 mol, bezogen auf 1 mol eingesetzten Alkohols, eventuell zusammen mit Stickstoff in einer Menge von bis zu 99 Vol.-% des Gesamtgasgemisches, über den auf 225 bis 500°C gehaltenen Katalysator leitet, wobei gegebenenfalls man den ersten Anteil an flüchtiger Phosphorverbindung dem gasförmigen Ausgangsgemisch zusetzt und mindestens einen weiteren Anteil in die Katalysatorschüttung nach dem Hot Spot im Bereich von 1 bis 35 % der Gesamthöhe der Schüttung zugegeben wird oder wobei man gegebenenfalls den ersten Anteil an flüchtiger Phosphorverbindung dem gasförmigen Ausgangsgemisch zusetzt und mindestens einen weiteren Anteil in die Katalysatorschüttung nach dem Hot Spot im Bereich von 1 bis 35 % der Gesamthöhe der Schüttung zugibt.

Das den Reaktor verlassende Gasgemisch wird üblicherweise mit Wasser ausgewaschen.

Man kann die Phosphorverbindung als Lösung in Wasser, Alkohol, bevorzugt dem eingesetzten Alkohol, oder geeigneten Lösungsmitteln, wie zum Beispiel Ethern, in flüssiger oder durch Verdampfen der Lösung in gasförmiger Form oder aber in Form der reinen gasförmigen Phosphorverbindung zugeben, wobei die Zugabe als verdampfte Lösung oder in reiner gasförmiger Form bevorzugt ist.

Das nach dem erfindungsgemäßen Verfahren aus Ethylenglykol erhaltene Glyoxal, das direkt in der handelsüblichen Form einer 40 gew.-%igen wässrigen Lösung erhalten werden kann, zeichnet sich durch eine hohe Reinheit aus, die auch bei langem Betriebszeitraum unverändert erhalten bleibt. Durch das erfindungsgemäße Verfahren wird Glyoxal in hohen Ausbeuten bei langen Katalysatorstandzeiten erhalten.

Das erfindungsgemäße Verfahren wird anhand der nachfolgenden Beispiele näher erläutert.

**Beispiel 1**

In einem Rohrreaktor aus Titan mit einem inneren Durchmesser von 20 mm wurden Katalysatorformkörper aus im wesentlichen phosphorfreien Kupferkatalysator aus (SF-Cu DIN 1708 Werkstoffnummer 2.0090) und Katalysatorformkörper aus einer Kupfer-Phosphorlegierung mit einem Phosphorgehalt von 1000 bei einer Katalysatorhöhe

von 300 mm (Katalysatorvolumen 95 ml) so strukturiert eingebaut, daß die oberen 10 cm der Katalysatorschüttung nach dem Reaktorkopf aus dem im wesentlichen phosphorfreien Katalysator und die übrige Schüttung aus (10 bis 30 cm nach dem Reaktorkopf) der phosphorhaltigen Kupfer-Phosphor-Legierung bestand. Man leitete pro Stunde ein Synthesegemisch bestehend aus 130 Nl Stickstoff und Luft und 12,4 g Ethylenglykol durch den Rohrreaktor. Die Reaktortemperatur wurde mit Hilfe einer Salzschmelze auf 360°C angestellt.

Die GHSV (gas hourly space velocity), die definiert ist als

$$GHSV = Gasvolumen/Katalysatorvolumen$$

betrug 1500 $h^{-1}$. Die LHSV (liquid hourly space velocity), die definiert ist als

$$LHSV = Flüssigkeitsvolumen / Katalysatorvolumen$$

betrug 0,12 $h^{-1}$. Die Verweilzeit, definiert als Quotient aus dem Katalysatorvolumen und der Gasmenge war 2,4 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ sowie nicht umgesetztes $O_2$ verblieben im Abgas und wurden in der Gasphase analysiert.

Das nach einer Laufzeit von 10 Tagen bei unterschiedlichen Umsätzen erhaltene Versuchsergebnis ist in der folgenden Tabelle 1 zusammengestellt.

**Beispiel 2**

Die Katalysatorschüttung des im Beispiel 1 beschriebenen Rohrreaktors wurde so strukturiert, daß die oberen 10 cm nach dem Reaktorkopf aus Formkörpern aus dem im wesentlichen phosphorfreien Kupferkatalysator SF-Cu und die übrige Schüttung (10 bis 30 cm nach dem Reaktorkopf) aus einer Mischung im Gewichtsverhältnis 1:10 von Formkörpern einer Kupfer-Phosphorlegierung mit einem Phosphorgehalt von 10.000 pm und Formkörpem aus dem im wesentlichen phosphorfreien Katalysator SF-Cu bestand.

Analog Beispiel 1 wurden 12,4 g/h Ethylenglykol und 130 NL/h eines Gemisches aus Stickstoff und Luft durch den Rohrreaktor geleitet.

Das nach einer Laufzeit von 10 Tagen bei unterschiedlichen Umsätzen erhaltene Versuchsergebnis ist in der folgenden Tabelle 1 zusammengestellt.

**Beispiel 3**

Der in Beispiel 1 beschriebene Rohrreaktor wurde über die gesamte Füllhöhe von 300 mm mit einer aus einem Gemisch aus Katalysatorformkörpem aus einer Kupfer-Phosphorlegierung mit einem Phosphorgehalt von 10.000 ppm und Formkörpem aus dem im wesentlichen phosphorfreien Kupferkatalysator SF-Cu im Gewichtsverhältnis 1:5 gefüllt.

Analog Beispiel 1 wurden 12,4 g/h Ethylenglykol und 130 NL/h eines Gemisches aus Stickstoff und Luft durch den Rohrreaktor geleitet.

Das nach einer Laufzeit von 10 Tagen bei unterschiedlichen Umsätzen erhaltene Versuchsergebnis ist in der folgenden Tabelle 1 zusammengefaßt.

**Beispiel 4**

Der in Beispiel 1 beschriebene Rohrreaktor wurde über die gesamte Füllhöhe von 300 mm mit einer aus einem Gemisch aus Formkörpern einer Kupfer-Phosphorlegierung mit einem Phosphorgehalt von 10.000 ppm und Formkörpem aus dem im wesentlichen phosphorfreien Kupferkatalysator SF-Cu im Gewichtsverhältnis 1:10 gefüllt.

Analog Beispiel 1 wurden 12,4 g/h Ethylenglykol und 130 NL/h eines Gemisches aus Stickstoff und Luft durch den Rohrreaktor geleitet.

Das nach einer Laufzeit von 10 Tagen bei unterschiedlichen Umsätzen erhaltene Versuchsergebnis ist in der folgenden Tabelle 1 zusammengefaßt.

**Vergleichsbeispiel V1**

Der in Beispiel 1 beschriebene Rohrreaktor wurde über die gesamte Schütthöhe mit dem im wesentlichen

phosphorfreien Katalysatorformkörpern aus SF-Cu gefüllt.

Analog Beispiel 1 wurden 12,4 g/h Ethylenglykol und 130 NL/h eines Gemisches aus Stickstoff und Luft durch den Rohrreaktor geleitet.

Das nach einer Laufzeit von 10 Tagen bei unterschiedlichen Umsätzen erhaltene Besuchsergebnis ist in der folgenden Tabelle 1 zusammengefaßt.

**Vergleichsbeispiel V2**

Der in Beispiel 1 beschriebene Rohrreaktor wurde über die gesamte Schütthöhe mit Katalysatorformkörpern aus einer Kupfer-Phosphorlegierung mit einem Phosphorgehalt von 10.000 ppm gefüllt.

Analog Beispiel 1 wurden 12,4 g/h Ethylenglykol und 130 NL/h eines Gemisches aus Stickstoff und Luft durch den Rohrreaktor geleitet.

Das nach einer Laufzeit von 10 Tagen bei unterschiedlichen Umsätzen erhaltene Versuchsergebnis ist in der folgenden Tabelle 1 zusammengefaßt.

Tabelle 1

| Beispiel | Ethylenglykol Umsatz [Mol%] | Glyoxal Selektivität [%] | $CO/CO_2$ Selektivität [%] | Luft [Nl/h] |
|---|---|---|---|---|
| 1 | 95,6 | 79,5 | 16,0 | 38 |
| 1 | 97,4 | 75,6 | 18,5 | 40 |
| 1 | 98,2 | 73,1 | 19,6 | 40 |
| 1 | 99,5 | 67,4 | 24,5 | 42 |
| 2 | 91,5 | 81,4 | 12,3 | 34 |
| 2 | 96,5 | 80,7 | 15,4 | 36 |
| 2 | 98,4 | 79,1 | 21,3 | 38 |
| 2 | 99,0 | 74,5 | 27,8 | 40 |
| 3 | 96,7 | 72,1 | 19,3 | 40 |
| 3 | 97,4 | 68,9 | 21,5 | 41 |
| 3 | 97,8 | 67,3 | 23,5 | 42 |
| 4 | 96,4 | 71,0 | 19,5 | 34 |
| 4 | 97,9 | 68,1 | 23,1 | 35 |
| V1 | 95,3 | 70,5 | 19,4 | 37 |
| V1 | 96,7 | 69,3 | 20,5 | 36 |
| V1 | 98,8 | 60,0 | 36,3 | 38 |
| V1 | 99,6 | 54,9 | 41 | 40 |
| V2 | 95,2 | 58,1 | 25,2 | 22 |
| V2 | 96,9 | 55,7 | 27,3 | 24 |
| V2 | 98,2 | 51,4 | 31,6 | 26 |
| V2 | 99,5 | 49,7 | 34,4 | 28 |

**Beispiel 5**

In einem Rohrreaktor aus rostfreiem Stahl mit einem inneren Durchmesser von 54 mm wurden Katalysatorformkörper aus im wesentlichen phosphorfreien Kupferkatalysator SF-Cu (DIN 1708 Werkstoffnummer 2.0090) und Katalysatorformkörper aus einer Kupfer-Phosphorlegierung mit einem Phosphorgehalt von 10.000 ppm so eingebaut, daß von den 250 cm Katalysatorfüllhöhe (Katalysatorvolumen 5,7 l) die ersten 59 cm nach dem Reaktorkopf aus dem im wesentlichen phosphorfreien Kupferkatalysator und die übrige Schüttung (60 bis 250 cm) aus einer homogenen Mischung im Gewichtsverhältnis 1:10 von Formkörpern der Kupfer-Phosphorlegierung mit einem Phosphorgehalt von

10.000 ppm und Formkörpern des im wesentlichen phosphorfreien Kupferkatalysators SF-Cu besteht.

Man leitete pro Stunde ein Synthesegasgemisch bestehend aus 870 g Ethylenglykol, 1880 NL Luft und 80 NL Stickstoff durch den Rohrreaktor. Die Reaktortemperatur wurde mit Hilfe einer Salzschmelze auf 365°C eingestellt.

Die Gesamtgasmenge, bestehend aus Kreisgas und Synthesegas, betrug 9500 Nl/h. Die GHSV (gas hourly space velocity) betrug 1650 $h^{-1}$. Die LHSV (liquid hourly space velocity) betrug 0,13 $h^{-1}$. Die Verweilzeit war 2,2 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ sowie unumgesetztes $O_2$ verblieben im Abgas und wurden in der Gasphase analysiert.

Das nach einer Laufzeit von 10 Tagen erhaltene Versuchsergebnis ist in Tabelle 2 zusammengestellt.

**Beispiel 6**

In einem Rohrreaktor aus rostfreiem Stahl mit einem inneren Durchmesser von 54 mm wurden Katalysatorformkörper aus im wesentlichen phosphorfreien Kupferkatalysator SF-Cu (DIN 1708 Werkstoffnummer 2.0090) und Katalysatorformkörper aus einer Kupfer-Phosphorlegierung mit einem Phosphorgehalt von 1000 ppm so eingebaut, daß von den 250 cm Katalysatorfüllhöhe (Katalysatorvolumen 5,7 l) die ersten 16 cm nach dem Reaktorkopf aus dem im wesentlichen phosphorfreien Kupferkatalysator und die übrige Schüttung (17 bis 250 cm) aus Formkörpern der phosphorhaltigen Kupfer-Phosphorlegierung mit einem Phosphorgehalt von 1000 ppm besteht.

Man leitete pro Stunde ein Synthesegasgemisch bestehend aus 870 g Ethylenglykol, 1760 NL Luft und 80 NL Stickstoff durch den Rohrreaktor. Die Reaktortemperatur wurde mit Hilfe einer Salzschmelze auf 365°C eingestellt.

Die Gesamtgasmenge, bestehend aus Kreisgas und Synthesegas, betrug 9500 Nl/h. Die GHSV (gas hourly space velocity) betrug 1650 $h^{-1}$. Die LHSV (liquid hourly space velocity) betrug 0,13 $h^{-1}$. Die Verweilzeit war 2,2 s.

Das Reaktionsgas wurde nach Verlassen des Reaktors mit Wasser in Berührung gebracht, wobei die Reaktionsprodukte in der wässrigen Phase gelöst wurden. Die bei der Reaktion entstandenen Permanentgase CO und $CO_2$ sowie unumgesetztes $O_2$ verblieben im Abgas und wurden in der Gasphase analysiert.

Das nach einer Laufzeit von 10 Tagen erhaltene Versuchsergebnis ist in Tabelle 2 zusammengestellt.

**Vergleichsbeispiel V3**

Man verfuhr wie in Beispiel 5 beschrieben. Die Katalysatorschüttung bestand jedoch über die gesamte Schütthöhe aus Formkörpern des im wesentlichen phosphorfreien Kupferkatalysators SF-Cu. Die Produktaufarbeitung erfolgt wie in Beispiel 5 beschrieben. Das nach einer Laufzeit von 10 Tagen erhaltene Versuchsergebnis ist in Tabelle 2 zusammengestellt.

**Vergleichsbeispiel V4**

Man verfuhr wie in Beispiel V3 beschrieben. Zum Synthesegasgemisch wurden am Reaktorkopf vor der Katalysatorschüttung 0,3 ppm Phosphor (P), bezogen auf die eingesetzte Gewichtsmenge an Ethylenglykol, in Form von Triethylphosphat gegeben.

Die Produktaufarbeitung erfolgte wie in Beispiel 5 beschrieben. Das nach einer Laufzeit von 10 Tagen erhaltene Versuchsergebnis ist in Tabelle 2 zusammengestellt.

Die folgende Tabelle 2 stellt die mit dem erfindungsgemäßen Beispiel 2 den mit den nicht-erfindungsgemäßen Vergleichsbeispielen V3 und V4 erzielten Ergebnisse gegenüber.

| Beispiel | Ethylenglykol-Umsatz [Mol%] | Glyoxal Selektivität [%] | $CO/CO_2$ Selektivität [%] | Glykolaldehyd Ausbeute [mol%] | Formaldehyd Ausbeute [mol%] | Luft [Nl/h] |
|---|---|---|---|---|---|---|
| 5 | 98,2 | 80,9 | 14,0 | 1,73 | 2,2 | 1880 |
| 6 | 98,3 | 82,5 | 10,3 | 0,64 | 3,7 | 1760 |
| V3 | 98,2 | 75,1 | 15,2 | 0,8 | 5,9 | 1800 |
| V4 | 98,8 | 76,9 | 14,0 | 1,1 | 5,9 | 1720 |

**Patentansprüche**

1. Verfahren zur Herstellung von Carbonylverbindungen der Formel

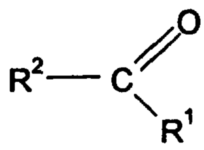

(I)

in der $R^1$ ein Wasserstoffatom oder einen Alkylrest mit 1 bis 3 C-Atomen, $R^2$ ein Wasserstoffatom, einen unsubstituierten oder mit $C_1$- bis $C_3$-Alkyl ein- bis dreifach substituierten $C_2$- bis $C_4$-Alkylenrest oder einem Rest der Formel

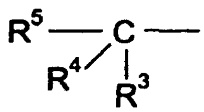

(II)

in der $R^3$ ein Wasserstoffatom oder zusammen mit $R^4$ ein Sauerstoffatom, $R^4$ den Rest $OR^6$ oder zusammen mit $R^3$ ein Sauerstoffatom, $R^5$ ein Wasserstoffatom, einen Alkylrest mit 1 bis 8 C-Atomen, einen unsubstituierten oder mit $C_1$- bis $C_3$-Alkyl ein- bis dreifach substituierten $C_2$- bis $C_4$-Alkylenrest oder einen Cyclohexyl- oder Cyclopentylrest und $R^6$ einen Alkylrest mit 1 bis 4 C-Atomen, der einen Cyclohexyloder Cyclopentylrest oder einen Rest der Formel $-CH_2-CHO$ oder $-CH_2-CH_2-O-CH2-CHO$ bedeuten, durch Gasphasenoxidation von Methanol oder Alkoholen der Formel

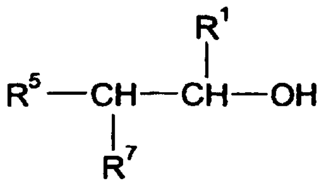

(III)

in der R1 und R5 die oben angegebene Bedeutung haben und R7 ein Wasserstoffatom oder einen Rest OR8 und R8 ein Wasserstoffatom, einen Alkylrest mit 1 bis 4 C-Atomen, einen Cyclohexyl- oder Cyclopentylrest oder einen Rest der Formel -CH2-CH2-OH oder -CH2-CH2-O-CH2-CH2-OH bedeutet, mit einem Sauerstoff enthaltenden Gas in Gegenwart von Kupfer und/oder Silber enthaltenden Katalysatoren und gegebenenfalls einer unter Reaktionsbedingungen flüchtigen Phosphorverbindung in einer Menge, so dass die Phosphormenge (berechnet als P), bezogen auf die Gewichtsmenge an eingesetztem Alkohol, bis zu 20 ppm beträgt, **dadurch gekennzeichnet, dass** eine Katalysatorschüttung aufgebaut aus einem Katalysatorsystem aus einem im wesentlichen phosphorfreien Kupfer und/oder Silber enthaltenden Katalysator mit einem Phosphorgehalt von 0 bis 400 ppm und mindestens einem phosphorhaltigen Kupfer und/oder Silber enthaltenden Katalysator mit einem Phosphorgehalt von 1000 bis 10000 ppm eingesetzt wird.

2. Verfahren nach Anspruch 1, **dadurch gekennzeichnet, dass** die gesamte Katalysatorschüttung aus einem Gemisch des im Wesentlichen phosphorfreien und des phosphorhaltigen Katalysators im Verhältnis 100:1 bis 1:100 aufgebaut ist.

3. Verfahren nach Anspruch 1, **dadurch gekennzeichnet, dass** die oberen 0,1 bis 50 % der Gesamthöhe der Katalysatorausschüttung nach dem Reaktorkopf aus dem im Wesentlichen phosphorfreien Katalysator und dass die verbleibende Katalysatorschüttung aus einer Mischung nach Anspruch 2 besteht.

4. Verfahren nach Anspruch 1, **dadurch gekennzeichnet, dass** die Katalysatorschüttung im Bereich der oberen 0,1 bis 50 % der Gesamthöhe der Katalysatorschüttung aus dem im Wesentlichen phosphorfreien Katalysator besteht und die restliche Katalysatorschüttung aus dem phosphorhaltigen Katalysator besteht.

**5.** Verfahren nach Anspruch 1, **dadurch gekennzeichnet, dass** eine flüchtige Phosphorverbindung am Reaktorkopf oder in die Katalysatorschüttung im Bereich der oberen 0,1 bis 50 % der Gesamthöhe der Katalysatorschüttung zugegeben wird.

**6.** Verfahren nach Anspruch 1, **dadurch gekennzeichnet, dass** die in Form der flüchtigen Phosphorverbindung zugegebene Phosphormenge (berechnet als P), bezogen auf die Gewichtsmenge an dem eingesetzten Alkohol, 0,05 bis 20 ppm beträgt.

**7.** Verfahren nach Anspruch 1, **dadurch gekennzeichnet, dass** die in Form der flüchtigen Phosphorverbindung zugegebene Phosphormenge in mindestens zwei Anteilen zugegeben wird, wobei

a) der erste Anteil mit dem gasförmigen Ausgangsgemisch vor der Katalysatorschüttung und
b) mindestens ein weiterer Anteil innerhalb der Katalysatorschüttung zugegeben wird.

**8.** Verfahren nach Anspruch 7, **dadurch gekennzeichnet, dass** mindestens ein weiterer Anteil b) in die Katalysatorschüttung im Bereich der oberen 0,1 bis 50 % der Gesamthöhe der Katalysatorschüttung zugegeben wird.

**9.** Verfahren nach Anspruch 1, **dadurch gekennzeichnet, dass** man Glyoxal aus Ethylenglykol herstellt.

## Claims

**1.** A process for preparing carbonyl compounds of the formula

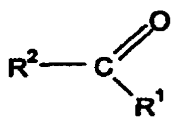

**(I)**

where $R^1$ is a hydrogen atom or an alkyl radical having from 1 to 3 carbon atoms, $R^2$ is a hydrogen atom, an unsubstituted or $C_1$-$C_3$-alkylmonosubstituted to -trisubstituted $C_2$-$C_4$-alkenyl radical or a radical of the formula

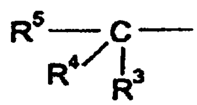

**(II)**

where $R^3$ is a hydrogen atom or together with $R^4$ is an oxygen atom, $R^4$ is the radical $OR^6$ or together with $R^3$ is an oxygen atom, $R^5$ is a hydrogen atom, an alkyl radical having from 1 to 8 carbon atoms, an unsubstituted or $C_1$-$C_3$-alkyl-monosubstituted to - trisubstituted $C_2$-$C_4$-alkenyl radical or a cyclohexyl or cyclopentyl radical and $R^6$ is an alkyl radical having from 1 to 4 carbon atoms, a cyclohexyl or cyclopentyl radical or a radical of the formula $-CH_2-CHO$ or $-CH_2-CH_2-O-CH_2-CHO$, by gas-phase oxidation of methanol or alcohols of the formula

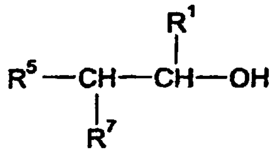

**(III)**

where $R^1$ and $R^5$ are as defined above and $R^7$ is a hydrogen atom or a radical $OR^8$ and $R^8$ is a hydrogen atom, an alkyl radical having from 1 to 4 carbon atoms, a cyclohexyl or cyclopentyl radical or a radical of the formula $-CH_2-CH_2-OH$ or $-CH_2-CH_2-O-CH_2-CH_2-OH$, by means of an oxygen-containing gas in the presence of copper- and/or silver-containing catalysts and in the presence or absence of a phosphorus compound which is volatile under the reaction conditions in such an amount that the amount of phosphorus (calculated as P) is up to 20 ppm, based on the total amount of alcohol used, wherein a catalyst bed made up of a catalyst system which comprises

an essentially phosphorus-free copper- and/or silver-containing catalyst having a phosphorus content of from 0 to 400 ppm and at least one phosphorus-containing copper- and/or silver-containing catalyst having a phosphorus content of from 1000 to 10 000 ppm is used.

**2.** A process as claimed in claim 1, wherein the total catalyst bed is made up of a mixture of the essentially phosphorus-free catalyst and the phosphorus-containing catalyst in a ratio of from 100:1 to 1:100.

**3.** A process as claimed in claim 1, wherein the upper 0.1 - 50% of the total height of the catalyst bed after the head of the reactor consists of the essentially phosphorus-free catalyst and the remainder of the catalyst bed consists of a mixture as claimed in claim 2.

**4.** A process as claimed in claim 1, wherein the catalyst bed in the region of the upper 0.1 - 50% of the total height of the catalyst bed consists of the essentially phosphorus-free catalyst and the remainder of the catalyst bed consists of the phosphorus-containing catalyst.

**5.** A process as claimed in claim 1, wherein a volatile phosphorus compound is introduced at the head of the reactor or into the catalyst bed in the region of the upper 0.1 - 50% of the total height of the catalyst bed.

**6.** A process as claimed in claim 1, wherein the amount of phosphorus (calculated as P) introduced in the form of the volatile phosphorus compound is from 0.05 to 20 ppm, based on the weight of the alcohol used.

**7.** A process as claimed in claim 1, wherein the phosphorus introduced in the form of the volatile phosphorus compound is introduced in at least two parts, where

a) the first part is introduced with the gaseous starting mixture upstream of the catalyst bed and

b) at least one further part is introduced within the catalyst bed.

**8.** A process as claimed in claim 7, wherein at least one further part b) is introduced into the catalyst bed in the region of the upper 0.1 - 50% of the total height of the catalyst bed.

**9.** A process as claimed in claim 1, wherein glyoxal is prepared from ethylene glycol.

## Revendications

**1.** Procédé de préparation de composés carbonyle de formule

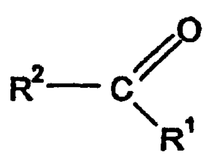

**(I)**

dans laquelle $R^1$ représente un atome d'hydrogène ou un groupe alkyle possédant 1 à 3 atomes de C, $R^2$ représente un atome d'hydrogène, un groupe alkylène en $C_2$ à $C_4$ non substitué ou substitué une à trois fois par un groupe alkyle en $C_1$ à $C_3$ ou un groupe de formule

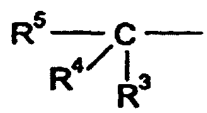

**(II)**

dans laquelle $R^3$ représente un atome d'hydrogène ou, conjointement avec $R^4$, un atome d'oxygène, $R^4$ représente le groupe $OR^6$ ou, conjointement avec $R^3$, un atome d'oxygène, $R^5$ représente un atome d'hydrogène, un groupe

alkyle possédant 1 à 8 atomes de C, un groupe alkylène en $C_2$ à $C_4$ non substitué ou substitué une à trois fois par un groupe alkyle en $C_1$ à $C_3$ ou un groupe cyclohexyle ou cyclopentyle et $R^6$ représente un groupe alkyle possédant 1 à 4 atomes de C, un groupe cyclohexyle ou cyclopentyle ou un groupe de formule $-CH_2-CHO$ ou $-CH_2-CH_2-O-CH_2-CHO$, au moyen d'une oxydation en phase gazeuse de méthanol ou d'alcools de formule

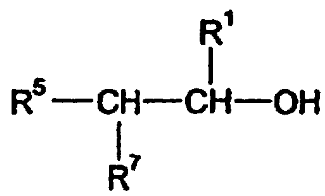

(III)

dans laquelle $R^1$ et $R^5$ ont les significations susmentionnées et $R^7$ représente un atome d'hydrogène ou un groupe $OR^8$ et $R^8$ représente un atome d'hydrogène, un groupe alkyle possédant 1 à 4 atomes de C, un groupe cyclohexyle ou cyclopentyle ou un groupe de formule $-CH_2-CH_2-OH$ ou $-CH_2-CH_2-O-CH_2-CH_2-OH$, avec un gaz contenant de l'oxygène en présence de catalyseurs contenant du cuivre et/ou de l'argent et éventuellement d'un composé du phosphore volatil dans les conditions de la réaction dans une quantité telle que la quantité de phosphore (exprimée en P), par rapport à la quantité en poids d'alcool mis en oeuvre, va jusqu'à 20 ppm, **caractérisé en ce que** l'on met en oeuvre un lit de catalyseur formé d'un système de catalyseur composé d'un catalyseur contenant du cuivre et/ou de l'argent et essentiellement exempt de phosphore avec une teneur en phosphore allant de 0 ppm à 400 ppm et d'au moins un catalyseur contenant du cuivre et/ou de l'argent et contenant du phosphore avec une teneur en phosphore allant de 1000 ppm à 10 000 ppm.

**2.** Procédé selon la revendication 1, **caractérisé en ce que** la totalité du lit de catalyseur est formée d'un mélange du catalyseur essentiellement exempt de phosphore et du catalyseur contenant du phosphore dans un rapport de 100:1 à 1:100.

**3.** Procédé selon la revendication 1, **caractérisé en ce que** les 0,1% à 50% supérieurs de la hauteur totale du lit de catalyseur se composent, après la tête du réacteur, du catalyseur essentiellement exempt de phosphore et que le lit de catalyseur restant se compose d'un mélange selon la revendication 2.

**4.** Procédé selon la revendication 1, **caractérisé en ce que** le lit de catalyseur dans la zone des 0,1% à 50% supérieurs de la hauteur totale du lit de catalyseur se composent du catalyseur essentiellement exempt de phosphore et le lit de catalyseur restant se compose du catalyseur contenant du phosphore.

**5.** Procédé selon la revendication 1, **caractérisé en ce que** l'on ajoute un composé volatil du phosphore dans la tête du réacteur ou dans le lit de catalyseur dans la zone des 0,1% à 50% supérieurs de la hauteur totale du lit de catalyseur.

**6.** Procédé selon la revendication 1, **caractérisé en ce que** la quantité de phosphore (exprimée en P) ajoutée sous la forme du composé volatil du phosphore va, par rapport à la quantité en poids de l'alcool mis en oeuvre, de 0,05 ppm à 20 ppm.

**7.** Procédé selon la revendication 1, **caractérisé en ce que** la quantité de phosphore ajoutée sous la forme du composé volatil du phosphore est ajoutée en au moins deux portions,

a) la première portion étant ajoutée avec le mélange de départ gazeux en amont du lit de catalyseur et
b) au moins une autre portion étant ajoutée à l'intérieur du lit de catalyseur.

**8.** Procédé selon la revendication 7, **caractérisé en ce que** l'on ajoute au moins une autre portion b) dans le lit de catalyseur dans la zone des 0,1% à 50% supérieurs de la hauteur totale du lit de catalyseur.

**9.** Procédé selon la revendication 1, **caractérisé en ce que** l'on produit du glyoxal à partir d'éthylèneglycol.